(19) Europäisches Patentamt
European Patent Office
Office européen des brevets

(11) Veröffentlichungsnummer: **0 013 865**
**A1**

(12) # EUROPÄISCHE PATENTANMELDUNG

(21) Anmeldenummer: **79810150.7**

(22) Anmeldetag: **12.11.79**

(30) Priorität: **17.11.78 CH 11829/78**

(43) Veröffentlichungstag der Anmeldung: **06.08.80 Patentblatt 80/16**

(84) Benannte Vertragsstaaten: **BE CH DE FR GB IT NL**

(51) Int. Cl.³: **C 07 D 211/02**, C 07 D 211/74

(71) Anmelder: **CIBA-GEIGY AG, Patentabteilung Postfach, CH-4002 Basel (CH)**

(72) Erfinder: **Orban, Ivan, Dr., Lehenmattstrasse 216, CH-4052 Basel (CH)**
Erfinder: **Troxler, Eduard, Dr., St. Albanring 220, CH-4052 Basel (CH)**

(54) **Verfahren zur Herstellung von 2,2,6,6-Tetraalkyl-4-oxopiperidinen.**

㊼ Verfahren zur Herstellung von Piperidinen der Formel (I)

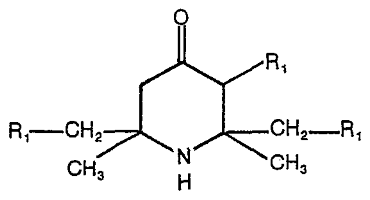

(I) ,

worin $R_1$ Wasserstoff oder $C_1$-$C_4$ Alkyl bedeutet, aus einem Keton der Formel (II) $R_1$-$CH_2$-C(O)-$CH_3$ (II), worin $R_1$ die oben angegebene Bedeutung hat, und Ammoniak, dadurch gekennzeichnet, daß man als Katalysator einen stark sauren Ionenaustauscher mit mittleren oder großen Makroporen verwendet.

EP 0 013 865 A1

CIBA-GEIGY AG
Basel (Schweiz)

3-12117/S/1+2

BEZEICHNUNG GEÄNDERT
siehe Titelseite

Neues Herstellungsverfahren

Die vorliegende Erfindung betrifft ein neues Verfahren zur Herstellung von 2,2,6,6-Tetraalkyl-4-oxopiperidinen.

Es sind verschiedene Verfahren zur Herstellung von 2,2,6,6-Tetraalkyl-4-oxopiperidinen, welche vor allem Bedeutung als Zwischenprodukte für die Herstellung von Lichtschutzmitteln gewonnen haben, bekannt. So wird z.B. im US-Patent 3,959,295 ein Verfahren beschrieben, welches 2,2,6,6-Tetramethyl-4-oxopiperidin ausgehend von Aceton und Ammoniak und in Gegenwart eines sauren Katalysators liefert. Bisher ist es allerdings nicht gelungen, ein Ionenaustauscher-katalysiertes Verfahren zu finden.

Die Vorteile, welche eine Ionenaustauscher-Katalyse mit sich bringen würde, wie z.B. deren Wirtschaftlichkeit durch wiederholten Einsatz, deren Umweltfreundlichkeit und leichte Trennbarkeit vom Endprodukt; sowie die gute Lagerstabilität der Produkte, da diese nicht mit löslichem Katalysator verunreinigt sind, usw., sind dem Fachmann geläufig.

Bisherige Versuche, Aceton und Ammoniak Ionenaustauscherkatalysiert umzusetzen, lieferten allerdings lediglich 2,2,4,4,6-Pentamethyl-2,3,4,5-tetrahydropyrimidin: Solche Verfahren mit sauren und basischen Ionenaustauschern als Katalysatoren sind z.B. im US-Patent 3,904,625 beschrieben worden.

Es wurde nun überraschend gefunden, dass Ionenaustauscher-katalysierte Reaktionen zwischen Ketonen und Ammoniak glatt 2,2,6,6-Tetraalkyl-4-oxopiperidine liefern, falls stark saure Ionenaustauscher mit bestimmtem Matrix-Aufbau verwendet werden.

Die vorliegende Erfindung betrifft ein Verfahren zur Herstellung von Piperidinen der Formel (I)

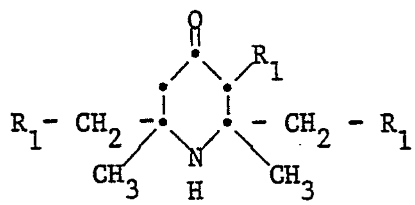

(I) ,

worin $R_1$ Wasserstoff oder $C_1$-$C_4$ Alkyl bedeutet, aus einem Keton der Formel (II) $R_1$-$CH_2$-C(O)-$CH_3$, worin $R_1$ die oben angegebene Bedeutung hat, und Ammoniak, dadurch gekennzeichnet, dass man als Katalysator einen stark sauren Ionenaustauscher mit mittleren oder grossen Maschen oder mit grossen Makroporen verwendet.

In den Formeln (I) und (II) bedeutet $R_1$ als $C_1$-$C_4$ Alkyl z.B. n-Butyl, t.-Butyl, n-Propyl, Aethyl oder bevorzugt Methyl. Insbesondere bedeutet $R_1$ allerdings Wasserstoff.

Stark saure Kationentauscher werden überwiegend auf Basis von Styrol hergestellt, wobei als Vernetzer z.B. Divinylbenzol zur Anwendung gelangt. Diese Copolymerisate werden mit den üblichen Sulfonierungsmitteln sulfoniert. Um zu dem für die vorliegende Erfindung wesentlichen Aufbau der Ionentauscher-Matrix zu gelangen, können grundsätzlich zwei Methoden angewendet werden:

a) Werden bei der Copolymersation vom Styrol und Divinylbenzol (DVB) die Anteile DVB niedrig gehalten, erhält man Copolymere mittlerer oder niedriger Vernetzung, d.h. die Maschen des dreidimensionalen Netzwerkes sind mittel oder gross. Als mittlere und grosse Maschen werden solche mit einer mittleren Weite von 10 - 20 Å bezeichnet. Für die vorliegende Erfindung werden solche mit grossen Maschen bevorzugt, d.h. solche mit Weiten von 12 - 20 Å. Solche Harze sind unter

dem Namen Gel-Harze bekannt geworden.

b) Werden bei der Copolymerisation von Styrol und DVB Intertmaterialien mitverwendet, und diese nach erfolgter Polymerisation z.B. durch Verdampfen oder Elution entfernt gelangt man zu sogenannten makroporösen Harzen. Die besten Resultate für die vorliegende Reaktion wurden mit grossen Poren erziehlt, deren mittlerer Porendurchmesser über ca. 500 Å, vorzugsweise über 600 Å liegt. Während dieser minimale mittlere Porendurchmesser ein erfindungswesentliches Merkmal darstellt, ist das Verwenden von Harzen mit grösseren Poren immer möglich. So sind zur Zeit Ionenaustauscher mit mittleren Porendurchmessern von 1300 Å und mehr im Handel erhältlich. Besonders gebräuchlich sind Ionenaustauscher mit Porendurchmessern von ca. 500 - 800 Å.

Die Umsetzung von Ammoniak mit den Ketonen der Formel II erfolgt auf an sich bekannte Weise, z.B. durch Einleiten von Ammoniak in das Keton in Gegenwart des Katalysators, bei Temperaturen von 5 bis 90°C, vorzugsweise 40 bis 75°C. Die Reaktion kann batchweise oder in einer für Ionenaustauscher besonders bevorzugten Variante auch kontinuierlich durchgeführt werden, wobei beispielsweise in einer mit dem erfindungsgemäss zu verwendenden Katalysator gefüllten Kolonne das Keton und Ammoniak in Kontakt gebracht werden. Dabei kann überschüssiges Keton laufend recyclisiert werden. Die wesentlichen Vorteile der kontinuierlichen Arbeitsweise sind die bessere Raum-Zeit-Ausbeute, erhöhte Betriebssicherheit durch erhebliche Reduktion der in der Anlage vorhandenen Acetonmenge, sowie kleinere Arbeitsintensität (z.B. durch Aufbereitung des Ketons, usw.).

Bei der erfindungsgemäss katalysierten Reaktion ist die Nebenproduktebildung besonders klein.

Das Mol-Verhältnis von Keton zu Ammoniak in dieser Reaktion beträgt mindestens 2:1, vorzugsweise 4:1. Der Ketonüberschuss kann jedoch durchaus bis 50:1 betragen. Es hat sich gezeigt, dass die Strömungsgeschwindigkeit praktisch keinen Einfluss auf die Umsatzgeschwindigkeit hat.

Die Reaktion kann auch bei leichterhöhtem Druck (z.B. bis 1 bar Ueberdruck) durchgeführt werden, was bei Temperaturen über dem Siedepunkt des verwendeten Ketons ohnehin der Fall ist. Bei batchweiser Arbeitsweise kann der Ueberdruck bis zu 30 bar, vorzugsweise bis zu 5 bar betragen.

Als Ketone der Formel II kommen beispielsweise n-Butylmethylketon, n-Propylmethylketon, bevorzugt Aethylmethylketon und insbesondere Aceton in Frage. Es ist auch möglich, Kondensationsprodukte dieser Ketone mit sich selbst oder mit Ammoniak einzusetzen. Dies hat vor allem für Aceton Bedeutung. So kann Aceton ganz oder teilweise durch ein saures Kondensationsprodukt mit sich selbst, wie Diacetonalkohol, Mesityloxid oder Phoron und ferner durch ein saures Kondensationsprodukt von Aceton mit Ammoniak, wie Diacetonamin, Triacetondiamin oder Acetonin ersetzt werden. Verwendet man anstelle des Acetons Acetonin, so kann auf Ammoniak ganz oder teilweise verzichtet werden, da dieses das erfindungsgemäss zu verwendende Ammoniak intramolekular bereits enthält. Selbstverständlich sind auch Mischungen mehrerer Kondensationsprodukte verwendbar.

Die Isolierung der erfindungsgemäss hergestellten alkylierten 4-Oxopiperidine erfolgt auf an sich bekannte Weise, z.B. durch Kristallisation und Filtration des Piperidinhydrats; oder durch Destillation des Produktes. Die zuletzt erwähnte Destillationsvariante kann ebenfalls kontinuierlich erfolgen.

<u>Beispiel 1</u>: Durch einen Reaktor von 83 cm Höhe und 6,5 cm Durchmesser, gefüllt mit entwässertem sauren Ionenaustauscher Lewatit SPC 118 H®, (total 1018 g) in 480 g Aceton wurden 138 g (2,38 Mol) Aceton und 5,8 g (0,34 Mol) Ammoniak pro Stunde durchgeleitet. Die erste Kontaktstelle befand sich dabei im oberen 13 cm langen Teil, welcher eine Innentemperatur von 25 bis 40°C besass. Der untere 70 cm

lange Teil wurde bei einer Temperatur von 55 bis 56°C gehalten. Dabei wird das verbrauchte Aceton (ca. 69 g/h) laufend ersetzt, und das überschüssige Aceton wird abdestilliert und mittels einem Kondensator der Kolonne erneut zugeführt. Das entstandene Triacetonamin wird laufend destilliert und ergibt ein Destillat von 30,9 g/h.

Es ist auch möglich, das entstandene 2,2,6,6-Tetramethyl-4-oxopiperidin (Triacetonamin) anstatt durch Destillation z.B. als Hydrat durch Kristallisation zu isolieren.

Beispiel 2: Wird bei sonst gleicher Arbeitsweise wie in Beispiel 1 als Katalysator Lewatit SC 102/H® verwendet, kommt zu praktisch den gleichen Ergebnissen wie in Beispiel 1.

Beispiel 3: Wird bei sonst gleicher Arbeitsweise wie in Beispiel 1 als Katalysator Lewatit SC 104/H® verwendet, bildet sich bei nur wenig verringerter Ausbeute glatt Triacetonamin.

Beispiel 4: Wird bei sonst gleicher Arbeitsweise wie in Beispiel 1 anstelle von 138 g Aceton/h ein Keton-Gemisch bestehend aus 87 g (1,5 Mol) Aceton, 30 g (0,3 Mol) Mesityloxid und 17 g (0,14 Mol) Diacetonalkohol verwendet, erhält man etwa die gleichen Mengen an Triacetonamin wie in Beispiel 1.

Beispiel 5: Wird bei sonst gleicher Arbeitsweise wie in Beispiel 1 anstelle von 138 g Aceton/h eine Lösung von 23 g (0,15 Mol) Acetoninhydrat, 107 g (1,84 Mol) Aceton und 8 g (0,44 Mol) Wasser verwendet, so erhält man Triacetonamin in gleichen Ausbeuten wie in Beispiel 1.

Beispiel 6: Die in Beispiel 1 beschriebene Reaktion wurde bei Strömungsgeschwindigkeiten von 12 $cm/h/cm^2$ und bei 500 $cm/h/cm^2$ durchgeführt. Beide Versuche ergaben nach gleicher Laufzeit praktisch identische Umsätze.

Beispiel 7: Wird bei sonst gleicher Arbeitsweise wie in Beispiel 1 als Katalysator Amberlist 15® verwendet, erhält man Triacetonamin in gleicher Ausbeute wie in Beispiel 1.

Beispiel 8: Wird bei sonst gleicher Arbeitsweise wie in Beispiel 1 als Katalysator Amberlite 200® verwendet, erhält man Triacetonamin in gleicher Ausbeute wie in Beispiel 1.

Beispiel 9: Die im Beispiel 1 beschriebene Versuchsanordnung wurde wie folgt variiert: Aceton und Ammoniak wurden in den im Beispiel 1 angegebenen Mengen von unten in einen Reaktor eingeleitet. Dabei beträgt die Temperatur im untersten Reaktorteil 20 - 65°C im oberen längeren Teil 65°C. Dabei entsteht Triacetonamin in praktisch gleicher Menge wie in Beispiel 1.

Beispiel 10: Ersetzt man in Beispiel 1 den Katalysator durch Duolite® (org. Ionenaustauscher, schwach sauer, makroporös) durch Amberlite IRC-50® (org. Ionenaustauscher, schwach sauer, Gel) durch Amberlite IR-4B® (org. Ionenaustauscher, schwach basisch, Gel), oder durch Amberlite IRA-900® (org. Ionenaustauscher, stark basisch, makroporös) erhält man praktisch kein Triacetonamin. Die Umsetzung von Aceton und Ammoniak liefert lediglich 2,2,4,4,6-Pentamethyl-2,3,4,5-tetrahydropyrimidin.

Patentansprüche

1. Verfahren zur Herstellung von Piperidinen der Formel (I)

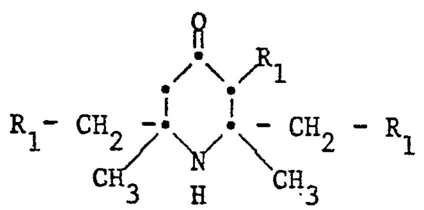

(I) ,

worin $R_1$ Wasserstoff oder $C_1$-$C_4$ Alkyl bedeutet, aus einem Keton der Formel (II) $R_1$-$CH_2$-C(O)-$CH_3$ (II), worin $R_1$ die oben angegebene Bedeutung hat, und Ammoniak, dadurch gekennzeichnet, dass man als Katalysator einen stark sauren Ionenaustauscher mit mittleren oder grossen Maschen oder mit grossen Makroporen verwendet.

2. Verfahren nach Anspruch 1, dadurch gekennzeichnet, dass in den Formeln (I) und (II) $R_1$ Wasserstoff oder Methyl bedeutet.

3. Verfahren nach Anspruch 1, dadurch gekennzeichnet, dass in den Formeln (I) und (II) $R_1$ Wasserstoff ist.

4. Verfahren nach Anspruch 3, dadurch gekennzeichnet, dass man das Keton der Formel $CH_3$-C(O)-$CH_3$ (Aceton) ganz oder teilweise mit Diacetonalkohol und/oder Mesityloxid, Phoron, Diacetonamin, Triacetondiamin oder Acetonin ersetzt.

5. Verfahren nach Anspruch 4, dadurch gekennzeichnet, dass man das Aceton durch Acetonin ersetzt, und da das erfindungsgemäss zu verwendende Ammoniak im Acetonin enthalten ist, auf weiteren Ammoniakzusatz verzichtet.

6. Verfahren nach Anspruch 1, dadurch gekennzeichnet, dass man ein Gelharz mit der Maschengrösse 10 bis 20 Å verwendet.

7. Verfahren nach Anspruch 1, dadurch gekennzeichnet, dass man ein makroporöses Harz mit einem Porendurchmesser von mindestens 500 Å verwendet.

8. Verfahren nach Anspruch 1, dadurch gekennzeichnet, dass man eine kontinuierliche Arbeitsweise wählt.

9. Verfahren nach Anspruch 1, dadurch gekennzeichnet, dass man einen der in den Beispielen 1 bis 3, 7 oder 8 beschriebenen Katalysatoren verwendet.

Europäisches Patentamt

# EUROPÄISCHER RECHERCHENBERICHT

0013865 Nummer der Anmeldung

EP 79 81 0150

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int.Cl. 3) |
|---|---|---|---|
| | CHEMICAL ABSTRACTS, Band 86, Nr. 7, 14. Februar 1977, Zusammenfassung Nr. 43562C, Seite 552, Columbus, Ohio, US,<br>& SU - A - 520 357 (KARAKULEVA et al) (5.7.1976)<br>* Zusammenfassung *<br>-- | 1,3-5 | C 07 D 211/02<br>211/74 |
| D | US - A - 3 904 625 (B.A. OUDE ALINK)<br>* Seite 2, Zeilen 52-59 *<br>-- | 1 | |
| D | US - A - 3 959 295 (ORBAN et al)<br>* Seiten 1,2 *<br>-- | 1 | RECHERCHIERTE SACHGEBIETE (Int. Cl. 3)<br>C 07 D 211/02<br>211/74 |
| PX | EP - A - 0 004 104 (CHEMISCHE WERKE HULS AKTIENG.)<br>* Seite 1, Zeilen 13-20; Seite 3, Zeilen 5-27 *<br>-- | 1 | |
| P | GB - A - 2 017 687 (CHIMOSA CHIMICA)<br>* Seite 2, Zeilen 29-36 *<br>-- | 1 | |
| A | FR - A - 2 203 813 (CIBA-GEIGY)<br>---- | | |

KATEGORIE DER GENANNTEN DOKUMENTE
- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 20-03-1980 | BRIGHENTI |